# EUROPEAN PATENT SPECIFICATION

(11) **EP 4 496 995 B1**
(45) Date of publication and mention of the grant of the patent: **24.12.2025**
(21) Application number: 23716913.1
(22) Date of filing: 23.03.2023
(51) Int. Cl.: G01N 21/64, G01N 33/543, G01N 21/77

(54) **A METHOD FOR DETECTING AN ANALYTE**
VERFAHREN ZUM NACHWEIS EINES ANALYTEN
PROCÉDÉ DE DÉTECTION D'UN ANALYSTE

(30) Priority: 23.03.2022 GB 202204107
(43) Date of publication of application: 29.01.2025
(73) Proprietor: Psyros Diagnostics Limited, Sandwich Kent CT13 9FE (GB)
(72) Inventor: ROSS, Steven Andrew, Aldington Kent TN25 7BB (GB); MCGETTRICK, Aileen Jane, Herne Bay Kent CT6 6HW (GB); RICHARDS, Julie, Faversham Kent ME13 7TE (GB); MONAGHAN, Paul Brendan, Sandwich Kent CT13 9FE (GB)
(74) Representative: Elkington and Fife LLP
(86) International application number: PCT/GB2023/050732
(87) International publication number: WO 2023/180747

(56) References cited:
- WO-A1-2020/260865
- MULVANEY ET AL: "Rapid, femtomolar bioassays in complex matrices combining microfluidics and magnetoelectronics", BIOSENSORS AND BIOELECTRONICS, ELSEVIER SCIENCE LTD, UK, AMSTERDAM , NL, vol. 23, no. 2, 16 September 2007 (2007-09-16), pages 191 - 200, XP022250735, ISSN: 0956-5663, DOI: 10.1016/J.BIOS.2007.03.029

## Description

The present invention relates to a method for detecting an analyte, and particularly to a method for detecting individual binding events due to the presence of an analyte in a sample.

There are many techniques available for the measurement of biologically relevant parameters in human samples, such as blood, plasma, serum, tissue etc. A common method is to use a capture reagent that binds to the target of interest, and a reporter reagent that has a label of some sort. The capture reagent can be bound to a solid phase, such as a microtitre plate, a bead or a membrane. When the sample is incubated with the capture reagent, the analyte binds to the capture reagent. The reporter reagent also binds to the analyte. Excess reporter can then be removed (by washing) and the quantity of reporter reagent can be measured, thus giving a measure of the amount of analyte present in the sample. There are many different variations on how these types of binding assay can be carried out. For example, the analyte can bind to the capture reagent first, then the reporter can be added in a separate step, or the analyte can bind to the reporter first, and then bind to the capture reagent.

There are a wide range of reagents that can be used as capture and reporter in binding assays of this type, including nucleic acids, carbohydrates, antigens, peptides, proteins and antibodies. There are also a wide range of target analytes, including peptides, proteins, antibodies, nucleic acids, cells, carbohydrates, small molecules, therapeutic drugs, drugs of abuse, steroids, hormones, lipids etc.

Assays that use antibodies are commonly termed immunoassays. Immunoassays can take a number of formats. For example, when a capture antibody is used to capture the analyte and a reporter antibody is used to generate a measurable signal, this is commonly called a sandwich immunoassay. Alternative formats are known, where the binding agent is attached to a solid phase and target analyte competes in solution with a labelled reagent that also binds to the binding agent. In the absence of analyte, a high signal is obtained, since high levels of the labelled reagent bind. In the presence of analyte, a fraction of the binding sites are blocked, thus less of the labelled reagent binds and the signal is reduced. These assays are commonly known as inhibition or competition assays. Several types of competition assay are known. For example, an antibody could be bound to the solid phase and labelled analyte (or analogue of the analyte) can compete for the binding sites on the antibody. Alternatively, an analogue of the analyte could be immobilised, and a labelled antibody could bind to this surface. In the presence of analyte in the sample, this will bind to the antibody in solution, preventing binding to the surface, and signal will be diminished.

There are many formats for assays, and many different types of label that can be employed. For example, assays can be heterogeneous, in that excess label is removed before the measurement takes place, for example by the use of wash steps. Removal of excess label can also be achieved by flowing sample and reporter past a capture area. This approach is used in immunochromatographic or lateral flow strips, which are used in rapid testing for infectious disease tests and pregnancy tests, for example. Alternatively, homogeneous assays are known, where the excess reporter is not removed. Homogeneous assays tend to rely on the close proximity of capture and reporter creating some form of signal. One example of a homogeneous assay is an agglutination assaym, where particles bind together in solution. The agglutinated particles cause scattering of light, which can be measured by turbidimetry or nephelometry. A further example of a homogeneous assay using particles is the luminescent oxygen channeling immunoassay (LOCI), described in further detail below.

A further example of a homogeneous assay is the fluorescence resonance energy transfer (FRET) assay, where the capture and reporter reagents are donor and acceptor fluorophores respectively, and excitation of the donor leads to energy transfer to the acceptor, followed by light emission.

One homogeneous assay format that functions in whole blood, without removal of cellular material is the pyro-optical immunoassay. Capture antibody is coated on to pyroelectric polyvinylidene PVDF sensor and carbon particles are used as the reporter. Signal is generated by illuminating the sample with light, causing localised heating of the particles. Those bound to the sensor transfer energy to the pyroelectric sensor, causing a thermal stress that is detected as an electrical signal. The more carbon bound, the greater the signal.

The label that is attached to the reporter binding agent can be an agent that absorbs light, such as a dye, gold particle or dyed latex microsphere. Larger particles can, in principle, absorb more light, and generate more signal. However, there is a size limit where particulate labels become impractical to use in assays, as described in more detail below. Luminescent labels are also known, such as fluorescent, chemiluminescent, bioluminescent and electrochemiluminescent labels. Luminescent labels have also been encapsulated in particles for certain types of assay. Amplification of the signal may also be performed by using enzymatic or catalytic reactions. Enzymes can be used to convert a substrate from a leuco dye to a coloured form, or into a fluorescent or luminescent form. It is common for the excess enzyme to be removed using wash steps before the substrate is added, so that signal is generated only by the enzyme that is bound specifically to the analyte.

Immunoassays that do not use labels are also known, such as assays that use surface plasmon resonance as the signal transduction method. However, label free assays tend to lack the sensitivity of assays that use labels to enhance the signal.

Further information on the field of immunoassays can be found in "The Immunoassay Handbook: 4th Edition: Theory and Applications of Ligand Binding, ELISA and Related Techniques", Ed. D. Wild, Elsevier Science, 2013.

All binding assays, including immunoassays, have limitations in terms of the minimum and maximum concentrations of analyte that can be reliably measured.

The signal maximum is generally limited by factors such as the total amount of capture antibody available to bind the analyte and the total amount of reporter antibody to generate signal. If the capture antibody is immobilised on a solid-phase, then the surface area of the solid-phase can limit the upper limit of detection. Additionally, some signal transduction techniques can be prone to saturation, such as colorimetric methods, depending on the pathlength that the light needs to take through the sample. Luminescent methods are less prone to saturation, as the gain on the detector can be attenuated to deal with higher levels of light emission. When all of the antibody binding sites in a heterogeneous assay are filled with analyte, then the maximum signal is reached and the system has saturated. The excess analyte will normally be removed in a wash step before the reporter is added. Homogeneous assays can also suffer from an effect known as high-dose hook, where the concentration of analyte is greater than the effective concentration of capture and/or reporter antibody. In this instance, at very high concentrations, all of the binding sites on the capture and the reporter can be blocked and the assay signal can be diminished, giving erroneous results.

The lower level of detection is governed by a number of different factors. Generally, all assays will be affected by attributes such as the quality of the antibodies being used (affinity and specificity) and cross-reactivity of the antibodies with related analytes. The lower limit of detection is also dependent upon the assay set-up and factors which affect the signal-to-noise ratio of the system design. For example, in a standard enzyme-linked immunosorbent assay (ELISA), capture antibody is coated onto the surface of a 96-well microtitre plate, and then sample is incubated in the wells, leading to the analyte being captured. The wells are washed, and then reporter is added in excess, binding to the captured analyte. Excess reporter is then washed away and substrate is added that can react with the enzyme, being converted into an active form. For example, a non-coloured leuco dye, such as 2,2'-azino-bis (3-ethylbenzothiazoline-6-sulphonic acid) (ABTS) can be converted by horseradish peroxidase into an oxidised green form in the presence of hydrogen peroxide. When the analyte is present in very low quantities (e.g. less than 1 picomolar), there will only be very small quantities of enzyme bound to the surface of the well. The ABTS reacts with the enzyme to generate the green form, then diffuses into the bulk of the fluid, generating a solution that is so dilute that it cannot be distinguished from background signal. Auto-conversion of substrate can also generate colour that interferes with the measurement. Similarly, other detection methods, such as fluorescence can suffer from interfering factors and auto-fluorescence of components in the sample or the reaction wells.

Another confounding factor in immunoassays can be non-specific binding of reporter reagents to the capture surface. For example, in the ELISA assay described above, the microtitre well is coated in a layer of protein, some of which can become de-natured during the coating process. It is not uncommon for reporter to bind to areas of the capture surface during the assay. If this reporter turns over substrate to contribute to the overall signal, it is not possible to distinguish the signal due to specifically bound reporter from that which is bound non-specifically. Non-specific binding can also be promoted by many of the components present in the original sample, which can bind to the capture surface during the initial incubation, modifying the surface properties of the capture layer, creating a surface that can bind the reporter. Minimising non-specific binding of reporter involves careful optimisation of all reagents and reaction conditions used during the assay, including antibodies, detergents, temperature and ionic strength.

Generally, the limit of detection of traditional immunoassays is around 0.1 picomolar to 1 nanomolar, depending upon the assay methodology. Developing assays with very low detection limits using traditional approaches often requires a great deal of optimisation, with stringent wash steps to reduce non-specific binding and maximise signal-to-noise. Additionally, the capture surface is often small in relation to the sample volume to ensure that the signal is high enough relative to the background.

One approach that has been used to circumvent issues relating to signal-to-noise and improve detection limits is to measure individual binding events, and to count these binding events as "on" or "off" events, if the measurement is above a localised threshold. In this way, much of the background noise can be eliminated. Analogies can be drawn with digitisation of audio or communication signals. These digital assays have been shown to reach detection limits that were previously unattainable using traditional analogue methods. For example, detection limits in the low femtomolar (10⁻¹⁵ mol/L) and even attomolar (10⁻¹⁸ mol/L) range have been reported. See for example "Evaluation of highly sensitive immunoassay technologies for quantitative measurements of sub-pg/mL levels of cytokines in human serum", Yeung et al., Journal of Immunological Methods, 2016, 437, 53 and "Digital Detection of Biomarkers Assisted by Nanoparticles: Application to Diagnostics", Cretich et al., Trends in Biotechnology, 2015, 33, 343.

The majority of labels/reporters used in assays (such as fluorophores, dyes etc.) are not visible individually using wide-field microscopy, even under high magnification, as their size is below the diffraction limit of visible light. Thus, the presence of these labels can only be measured as a bulk phenomenon, not by counting each label. In contrast, particulate labels, such as latex particles, can in theory be visualised by wide-field optical microscopy if they are above a certain size. Depending upon the optical set-up, it is possible to start visualising particles when they have a diameter of several hundred nanometres and above, depending on the numerical aperture and type of microscope.

However, using particles of this size as labels to monitor individual binding events on a capture surface (such as an antibody-antigen interaction) becomes impractical for a number of reasons. For example, particles of this size diffuse very slowly in comparison to other types of label, impairing the rate of the reaction at a planar surface. They also start to exhibit macroscopic buoyancy effects, and will sediment or float if the density of the particle is significantly different to the medium that they are contained in, which can also cause issues with assay format. Particles of this size are particularly prone to binding non-specifically to surfaces, causing high background, which is difficult to remove. Finally, excess particles must be removed, requiring wash steps. However, large particles start to suffer from shear effects in the presence of fluid flow, where the shear force on the particle becomes greater than the rupture strength of the antibody-antigen interaction (approximately 60-250 pN), leading to particles being washed away (see "Rapid Femtomolar Bioassays in Complex Matrices Combining Microfluidics and Magnetoelectronics", Mulvaney et al., Biosensors and Bioelectronics, 2007, 23, 191).

Examples of digital assays include the Quanterix Single Molecule Array (SIMOA) system and the Singulex Single Molecule Counting (SMC) system.

The Quanterix SIMOA system uses antibody-coated paramagnetic beads to capture analyte from solution. The magnetic beads are then washed, and reporter antibody labelled with enzyme is added. The quantity of beads is sufficient that the probability of having more than one analyte and reporter per bead is minimised. The beads are washed again, then loaded into an array of microwells that can only hold one bead per well. The volume of the microwells is on the femtolitre scale. If the bead has enzyme attached, then fluorogenic substrate in the well is turned over. The small dimensions of the well prevent the fluorescent product from becoming too diffuse. Each well is then counted as an "on" or "off" event if the fluorescence is above a threshold value.

The SMC system is used in the Singulex Clarity instrument and also in the Erenna and SMCxPRO systems from Merck Millipore. The fundamental measurement technique in all three systems is the same. Magnetic beads, coated in capture antibody, are used to capture the target analyte in a sandwich assay. Fluorescently labelled reporter antibody also binds to the bead, in the presence of analyte. The beads are pulled down with a magnet and excess fluorescently-tagged reporter is washed away. An eluting buffer is then added that causes dissociation of the sandwich complex, which is then transferred to a measurement vessel. The presence of the fluorescent tag is then measured using a confocal fluorescent microscope that sequentially interrogates small volumes of the sample to determine if the fluorescent tag is present or not. If the signal for each individual measurement is above a threshold value, this counts as an "on" event for that measurement.

Several independent academic reviews of high-sensitivity immunoassays have highlighted that the digital approach to immunoassays allows unprecedented improvements to detection limits (see references Yeung and Cretich above).

The detection limit of the Quanterix and Singulex systems is dependent upon the volume of sample that is used in the assay. For a 10 microlitre serum or plasma sample, the theoretical limit would be the detection of a single binding event, corresponding to 1 molecule. However, in terms of molarity, this would correspond to 100,000 molecules per litre of sample, or 0.16 x 10⁻¹⁸ moles per litre (0.16 attomolar).

However, the Quanterix and Singulex systems described above are complex and cumbersome, each requiring a number of wash and transfer steps. Further, the assays can only be carried out for samples that do not contain cellular material and the systems require expensive instrumentation in order to achieve the performance that they offer. There therefore remains a need for simpler and more cost-effective high-sensitivity systems.

WO2020/260865 describes a digital assay method that uses a photosensitiser reagent to convert an optical component from a first optical state to a second optical state. However, there remains a need for the optimisation/enhancement of this conversion.

Accordingly, the present invention provides a method for detecting an analyte in a sample, the method comprising the steps of:
(i) providing a mixture comprising a sample and a reporter reagent to a device, wherein the reporter reagent comprises a photosensitiser, the device comprising a substrate having an optical component and a binding component, wherein the optical component and the binding component are attached to a surface of the substrate;
(ii) allowing a portion of the reporter reagent to bind to the surface of the substrate in proportion to the concentration of the analyte, by means of the binding component;
(iii) irradiating the device with electromagnetic radiation for absorption by the optical component and for absorption by the photosensitiser of the reporter reagent, such that the optical component absorbs electromagnetic radiation to generate an excited optical component, and the photosensitiser of the reporter reagent bound to the surface of the substrate absorbs electromagnetic radiation and interacts with the excited optical component to cause the excited optical component to change from a first optical state to a second optical state, thereby forming a set of local regions having the second optical state on the substrate; and
(iv) detecting the set of local regions having the second optical state on the substrate.

Thus, the present invention provides a method for detecting an analyte in a sample in which only the reporter reagent in the vicinity of the surface of the substrate results in a signal, the signal being a local region of the optical component in the second optical state. It is the set of local regions of the optical component in the second optical state that are detected. Therefore, the present invention simplifies digital detection of the analyte and facilitates homogeneous assays on a range of samples, including those containing cellular materials.

The inventors have surprisingly found that conversion of the optical component from the first optical state to the second optical state is more efficient when the optical component is also excited by electromagnetic radiation. Specifically, the conversion of the optical component occurs more rapidly when both the reporter reagent and the optical component are excited by electromagnetic radiation.

Additionally, the intensity of electromagnetic radiation required for conversion is reduced and it is possible to create discrete local regions having the second optical state that are larger in size, reducing the complexity of the optical set-up required to visualise these areas.

The present invention will now be described with reference to the drawings, in which:
Fig. 1 shows various components which may be used in the method of the present invention;
Fig. 2 shows a device in which a reporter reagent is bound to the surface of the substrate before irradiation;
Fig. 3 shows the device of Fig. 2 being irradiated;
Fig. 4 shows the device of Fig. 3 after irradiation;
Fig. 5 shows a representative example of a set of local regions having the second optical state on the substrate, visible as dark areas bleached in a fluorescent layer;
Fig. 6 shows an optical set-up for detection that allows two electromagnetic radiation sources to be focussed through an objective lens;
Fig. 7 shows a substrate and well created for the method of the present invention;
Fig. 8 shows a sample chamber created using the substrate and well of Fig. 7;
Fig. 9 shows the rate of spot formation in a streptavidin AlexaFluor 594 layer as a function of time and irradiation source as described in Example 4; and
Fig. 10 shows the rate of spot formation in a streptavidin Oregon Green 488 layer as a function of time and irradiation source as described in Example 5.

The method of the present invention is used for detecting an analyte in a sample (which may be via the detection of a complex or derivative of the analyte).

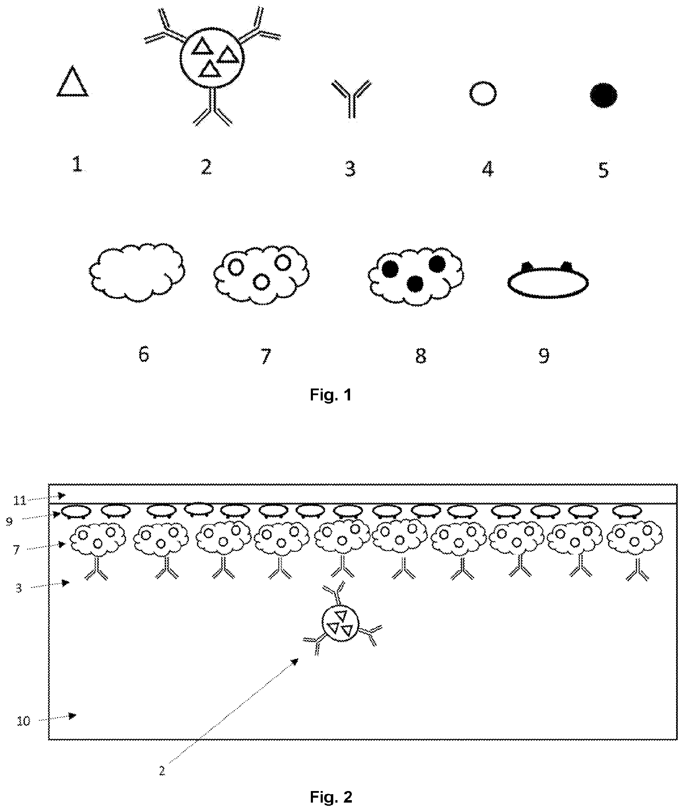

The components of Fig. 1 are: photosensitiser 1; antibody-coated latex particle infused with photosensitiser 2 (also referred to below as photosensitiser-labelled antibody 2); antibody 3; optical component in fluorescent state 4; optical component in non-fluorescent state 5; streptavidin 6, streptavidin labelled with optical component in fluorescent state 7 (also referred to below as streptavidin-dye conjugate 7); streptavidin labelled with optical component in non-fluorescent state 8; and biotinylated poly-lysine 9 (also referred to below as poly-lysine biotin conjugate 9).

Step (i) of the method of the present invention involves providing a mixture comprising a sample and a reporter reagent to a device, wherein the reporter reagent comprises a photosensitiser, the device comprising a substrate 11 having an optical component and a binding component, wherein the optical component and the binding component are attached to a surface of the substrate 11. The sample and the reporter reagent may be pre-mixed before adding the mixture to the device or the sample and the reporter reagent may be added sequentially to the device to form the mixture. The mixture may also contain additional reagents but preferably, the mixture consists of the sample and the reporter reagent.

By way of an explanation of the principle underlying the present invention, Fig. 2 shows a device in which a reporter reagent is bound to the surface of the substrate before irradiation. The device includes a substrate 11 and a sample chamber 10 for holding a sample containing an analyte dissolved or suspended therein. The substrate may be any substrate that allows detection of the set of local regions having the second optical state on the substrate. Preferably, the substrate is planar. Preferably, the substrate is a transparent substrate and more preferably, the substrate is glass or plastic.

The substrate 11 has antibody 3 bound to streptavidin-dye conjugate 7 attached to a surface of the substrate 11 via poly-lysine biotin conjugate 9. The dye acts as the optical component and the antibody acts as the binding component. Poly-lysine biotin conjugate 9 is an inert macromolecule that facilitates attachment of the optical and binding components to the surface of the substrate 11.

Although the optical and binding components are shown in this way, any technique for holding the optical and binding components proximal to the surface of the substrate 11 is applicable. For example, the optical and binding components may be a single reagent. The optical component may also be encapsulated within a polymer layer which is coated onto the surface of the substrate 11 and the binding component attached to the polymer layer. The polymer may be silicone, polystyrene or polyisobutylene, or any other suitable polymeric plastic that can be used to encapsulate the optical component.

Alternatively, a gel layer, e.g. a hydrogel layer may be impregnated with the optical component and the gel/hydrogel layer coated onto the surface of the substrate 11 and the binding component attached to the gel/hydrogel layer.

Step (ii) of the method of the present invention involves allowing a proportion of the reporter reagent to bind to the surface of the substrate in proportion to the concentration of the analyte, by means of the binding component. This may be achieved by allowing the device to stand for a period of time, for example 10 minutes.

In Fig. 2, antibody-coated latex particle infused with photosensitiser 2 is bound to the surface of the substrate 11 by means of antibody 3. For example, antibody 3 could be a mouse antibody and the antibody on the antibody-coated latex particle infused with photosensitiser 2 could be an anti-mouse antibody. Antibody-coated latex particle infused with photosensitiser 2 acts as the reporter reagent.

Although the reporter reagent is shown bound to the surface of the substrate 11 in this way, when analyte is present, the reporter reagent binds to the surface of the substrate in proportion to the concentration of the analyte. For example, if the binding component and the reporter reagent are antibodies, and the analyte is an antigen, the reporter reagent binds to the binding component via the analyte to form a so-called "sandwich" complex. Other binding events, such as antibody-hapten binding or nucleic acid binding are also possible.

All steps up until this point have taken place in the absence of light. Step (iii) of the method of the present invention involves irradiating the device with electromagnetic radiation for absorption by the optical component and for absorption by the photosensitiser of the reporter reagent, such that the optical component absorbs electromagnetic radiation to generate an excited optical component, and the photosensitiser of the reporter reagent bound to the surface of the substrate absorbs electromagnetic radiation and interacts with the excited optical component to cause the excited optical component to change from a first optical state to a second optical state, thereby forming a set of local regions having the second optical state on the substrate 11.

The electromagnetic radiation used to irradiate the device (typically termed "light") comprises the excitation wavelengths both of the optical component and the photosensitiser of the reporter reagent.

The use of electromagnetic radiation for absorption by the optical component and for absorption by the photosensitiser provides a significant advantage over previous assays, in which the device would only be irradiated with electromagnetic radiation for absorption by the photosensitiser, in that the change of the excited optical component from the first optical state to the second optical state happens more quickly and more efficiently and can also generate larger local regions in the second optical state. In this way, results of the assay can be obtained faster, and/or lower intensity light can be used to achieve the same results. Furthermore, a broadband light source can be used without requiring filters to remove unwanted parts of the electromagnetic spectrum, meaning simpler apparatus can be used.

Although not wishing to be bound by theory, it is believed that the photosensitiser of the reporter reagent bound to the surface of the substrate absorbs electromagnetic radiation and interacts with the excited state of the optical component more rapidly due to the optical component being in a higher energy state.

In a preferred embodiment, the electromagnetic radiation for absorption by the optical component and for absorption by the photosensitiser of the reporter reagent have the same wavelength. For example, the electromagnetic radiation used to irradiate the device may consist of the excitation wavelengths of both the optical component and the photosensitiser of the reporter reagent. The electromagnetic radiation used to irradiate the device may also consist of more wavelengths than the excitation wavelengths of the photosensitiser of the reporter reagent and of the optical component, for example broad-spectrum electromagnetic radiation.

When the electromagnetic radiation for absorption by the optical component and for absorption by the photosensitiser of the reporter reagent have the same wavelength, the device is irradiated with electromagnetic radiation for absorption by the optical component and for absorption by the photosensitiser of the reporter reagent simultaneously using one source of electromagnetic radiation.

Using the same wavelength of electromagnetic for absorption by the optical component and for absorption by the photosensitiser of the reporter reagent provides a simpler and more cost-effective method.

Alternatively, in a preferred embodiment, the electromagnetic radiation for absorption by the optical component and for absorption by the photosensitiser of the reporter reagent have different wavelengths. For example, the electromagnetic radiation may consist essentially of the excitation wavelengths both of the photosensitiser of the reporter reagent, and of the optical component. By "consist essentially of" it is meant as narrow window as possible around the required wavelength.

Using different wavelengths of electromagnetic radiation for absorption by the optical component and for absorption by the photosensitiser of the reporter reagent provides a more controllable method.

In this regard, the electromagnetic radiation may come from more than one source.

When two sources of electromagnetic radiation are used, one source for absorption by the optical component and the other source for absorption of the photosensitiser of the reporter reagent, the sources may be focused through an objective lens at the same time. Therefore, in a preferred embodiment, the electromagnetic radiation for absorption by the optical component and for absorption by the photosensitiser of the reporter reagent arrives at the device from one direction, more preferably through one objective lens.

The electromagnetic radiation for absorption by the optical component and for absorption by the photosensitiser of the reporter reagent may also arrive at the device from different directions. The electromagnetic radiation for absorption by the optical component may be focused through the objective lens, and the electromagnetic radiation for absorption by the photosensitiser may not be focused through the objective lens. Therefore, in a preferred embodiment, the electromagnetic radiation for absorption by the optical component and for absorption by the photosensitiser of the reporter reagent arrives at the device from different directions, more preferably the electromagnetic radiation for absorption by the optical component through an objective lens and the electromagnetic radiation for the photosensitiser does not arrive at the device through the objective lens.

When the electromagnetic radiation for absorption by the optical component and for absorption by the photosensitiser of the reporter reagent have different wavelengths, the device may be irradiated with electromagnetic radiation for absorption by the photosensitiser of the reporter reagent, and for absorption by the optical component, simultaneously. Alternatively, the device may be irradiated first with electromagnetic radiation for absorption by the photosensitiser of the reporter reagent and secondly with electromagnetic radiation for absorption by the optical component. Therefore, in a preferred embodiment, the device is irradiated with electromagnetic radiation for absorption by the optical component and for absorption by the photosensitiser of the reporter reagent simultaneously, or the device is irradiated first with electromagnetic radiation for absorption by the photosensitiser of the reporter reagent and secondly with electromagnetic radiation for absorption by the optical component.

In one embodiment, the device is irradiated first with electromagnetic radiation for absorption by the photosensitiser of the reporter reagent and secondly with electromagnetic radiation for absorption by the optical component. This ensures that the photosensitiser of the reporter reagent having absorbed electromagnetic radiation is able to interact with the excited optical component.

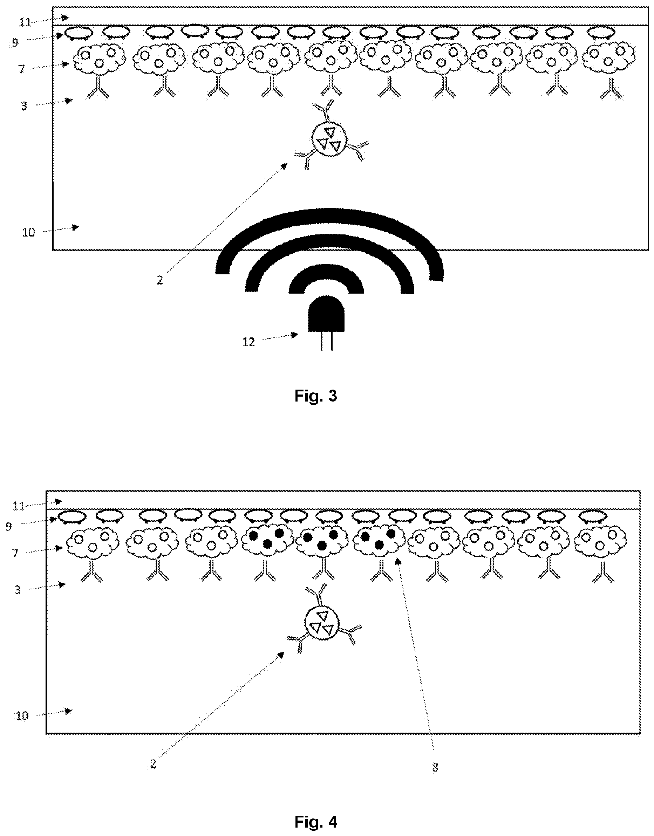

Fig. 3 shows the device of Fig. 2 being irradiated with electromagnetic radiation, preferably visible light. The light source may be, for example, LED 12. The light source illuminates the sample chamber 10 with light of the appropriate wavelength to excite the photosensitiser 2 and the dye component of streptavidin-dye conjugate 7. The wavelength depends on the photosensitiser and the dye. The device is typically irradiated for at least 10 seconds. Preferably, the device is irradiated with electromagnetic radiation for more than 1 second, more preferably at least 2 seconds, more preferably at least 5 seconds, more preferably at least 20 seconds and most preferably at least 30 seconds. This ensures that there is an irreversible optical change on the surface of the substrate, thus allowing long-lived and transient binding events to be distinguished.

Fig. 4 shows the device of Fig. 3 after irradiation. The photosensitiser 1 interacts with the irradiated dye optical component of the streptavidin-dye conjugate 7 to cause the dye to change from a fluorescent state to a non-fluorescent state. Streptavidin-dye conjugate in fluorescent state 7 becomes streptavidin-dye conjugate in non-fluorescent state 8. Only the dye that is in close proximity to photosensitiser 1 changes from the first optical state to the second optical state.

Alternative first and second optical states could include changes to light polarisation, fluorescence lifetime, refractive index, light scattering (including Raman scattering), phosphorescence and other optical effects.

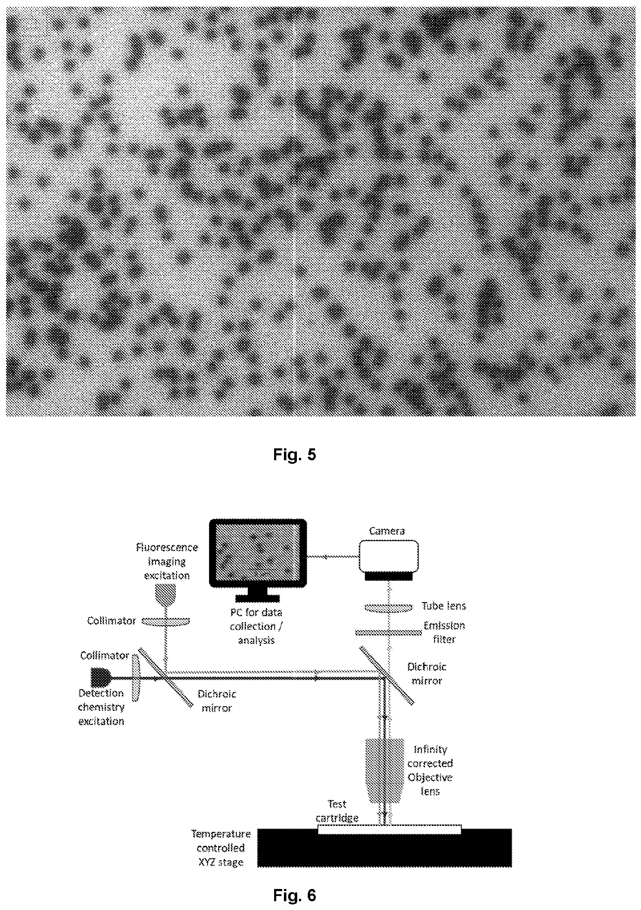

Although Figs. 2-4 illustrate one reporter reagent bound to the surface of the substrate 11, in reality multiple reporter reagents will bind to the surface of the substrate 11 to create more than one local region having the second optical state on the substrate. Using the components of Fig. 1, these locals regions having the second optical state on the substrate would be visible as discrete areas with the streptavidin-dye conjugate in non-fluorescent state 8. Such discrete areas can be seen in Fig. 5, which shows a representative example of a set of local regions having the second optical state on the substrate, visible as dark areas bleached in a fluorescent layer.

Any photosensitiser in the vicinity of the excited optical component interacts to cause the excited optical component to change from the first optical state to the second optical state. In this way, the photosensitiser of the reporter reagent bound to the surface of the substrate interacts with the excited optical component to cause the excited optical component to change from the first optical state to the second optical state, thereby forming a set of local regions having the second optical state on the substrate 11.

Figs. 2-4 also show the reporter reagent bound to the surface of the substrate 11 via the binding component. However, when measuring analyte concentration, the binding to the surface will be mediated by the analyte, for example in a "sandwich" assay.

Additionally, excess particles can be suspended in the sample that are not bound to the surface. If the sample is whole blood, then red cells will be suspended in the medium. Preferably, the substrate 11 forms the top of the sample chamber 10, allowing red blood cells to sediment away from the substrate 11.

A proportion of the reporter reagent binds to the surface of the substrate 11 by means of the binding component. The sample therefore contains bound reporter reagent and unbound reporter reagent free in solution. The depth of the sample chamber 10 is designed to minimise the diffusion path length of the reporter reagent, and allow equilibrium to be attained rapidly. Typically, the depth of the sample chamber is from 50 to 200 µm.

In the method of the present invention, the sample chamber 10 is filled with a sample containing an analyte. A reporter reagent such as photosensitiser-labelled antibody 2 is also added to the sample chamber 10. If a whole blood sample is used, the sample may also contain additional components such as red blood cells.

An equilibrium is then attained. A reporter reagent such as photosensitiser-labelled antibody 2 is bound to the surface of the substrate 11 in proportion to the concentration of analyte by means of a binding component such as antibody 3. An excess of reporter reagent is included so that a significant proportion of the analyte forms a sandwich complex. A proportion of the reporter reagent thus binds to the surface of the substrate 11 in proportion to the concentration of the analyte, by means of the binding component. The sample therefore contains bound reporter reagent and unbound reporter reagent free in solution.

After irradiation, the photosensitiser interacts with the excited optical component to cause the excited optical component to change from a first optical state to a second optical state. For example, streptavidin labelled with optical component in fluorescent state 7 becomes streptavidin labelled with optical component in non-fluorescent state 8. Only the optical component that is in close proximity to the photosensitiser changes from the first optical state to the second optical state.

The reporter reagent must be permanently bound to the surface of the substrate 11 during the entire period of irradiation in order to achieve complete conversion of the first optical state to the second optical state. If the reporter reagent is only transiently bound to the surface for a fraction of the irradiation period, then there is incomplete conversion to the second optical state, which can be detected by algorithms used to measure the size, shape and intensity of the discrete areas. Any unbound reporter reagent in solution does not cause the optical component to change significantly from the first optical state to the second optical state.

This provides a significant advantage over other digital assay methods in that it removes the need for washing steps. In this way, the method of the present invention is a homogeneous assay. In a conventional assay, the unbound reporter reagent must be separated from the bound reporter reagent before any measurement is taken since the unbound reporter reagent interferes with the signal generated by the bound reporter reagent. However, on account of the localised surface changes provided by the present invention, bound and unbound reporter reagent may be distinguished. Indeed, the ability to distinguish between reporter reagents proximal to the surface of the substrate 11 (i.e. bound) and reporter reagents in the bulk solution (i.e. unbound) is a particular advantage of the present invention. Preferably, steps (i) to (iii) take place in the absence of wash steps, i.e. the method is carried out without removing the sample from the substrate in steps (i), (ii) and (iii).

The photosensitiser of the reporter reagent bound to the surface of the substrate may interact directly with the optical component to cause the change (e.g. the photosensitiser is excited and transfers this energy directly to the excited optical component) or indirectly with the optical component to cause the change via an additional reagent (e.g. the photosensitiser is excited and transfers this energy to an additional component, which then transfers this energy to the excited optical component).

In a preferred embodiment, the photosensitiser of the reporter reagent absorbs electromagnetic radiation and interacts with a pre-activator reagent present in the mixture to generate an activator reagent which interacts with the excited optical component to cause the excited optical component to change from the first optical state to the second optical state. Therefore, the reporter reagent is capable of generating an activator reagent from a pre-activator reagent present in the mixture upon absorption of electromagnetic radiation, and the excited optical component is capable of changing from a first optical state to a second optical state on interaction with the activator reagent.

The pre-activator reagent may be present in the sample or the pre-activator reagent may be added to the mixture of the sample and the reporter reagent as an additional reagent. In a preferred embodiment, the pre-activator reagent is ground state triplet oxygen. In another preferred embodiment, the activator reagent is a reactive oxygen species (ROS). Preferably, the ROS is selected from a hydroxyl radical, superoxide, peroxide, organic peroxides, peroxynitrite, singlet oxygen and mixtures thereof. More preferably, the ROS is singlet oxygen. Singlet oxygen is the preferred activator reagent because it has a short half-life and finite diffusion path length (normally less than 1 micron under aqueous conditions).

It is believed that the photosensitiser absorbs the light to generate an excited state which can undergo intersystem crossing (ISC) with oxygen present in the sample and proximal to the reporter reagent to generate singlet oxygen. Singlet oxygen then goes on to interact with the excited optical component as discussed below. In a particularly preferred embodiment, the pre-activator reagent is ground state triplet oxygen and the activator reagent is singlet oxygen.

In the embodiment where a pre-activator reagent is converted into an activator reagent and if the activator reagent has a finite lifetime, there will be a short period of time post-irradiation of the photosensitiser where the activator reagent is still present in the vicinity of the binding event, depending upon the half-life of the activator reagent. Therefore, the irradiation of the photosensitiser and optical component may be slightly time shifted. At one extreme, the photosensitiser could be illuminated first to generate the activator reagent and then the optical component could be illuminated subsequently for the lifetime of the activator reagent.

Singlet oxygen has previously been used in immunoassays such as the luminescent oxygen channelling immunoassay (LOCI). The LOCI immunoassay is a homogenous non-digital assay using donor and acceptor beads that measures a bulk phenomenon. The donor beads generate singlet oxygen upon illumination at 680 nm, and the acceptor beads generate a chemiluminescent signal when activated by singlet oxygen. Binding of donor to acceptor beads is promoted by antibody-antigen binding. The reaction mixture is typically illuminated for 0.5 to 1.0 seconds and then the luminescence signal is measured for 0.5 to 1.0 seconds. Critically, the measurement takes place in the presence of all unbound donor and acceptor beads. Spatial separation of the beads minimises background signal; however, the LOCI assay cannot distinguish between long-lived and transient binding events, due to the short measurement time. The assay can achieve a detection limit of around 1-5 pg/mL for its most sensitive assays, for example interleukin 6 (IL-6) or thyroid stimulating hormone (TSH). The method of the present invention minimises background signal even further because the "donor" particles, the reporter reagent, needs to be in proximity to the surface of the substrate 11, rather than to particles in solution, in order for a signal to be detected, and must also be there for the duration of the illumination period in order to generate a signal that is above (or below) threshold criteria used in the image analysis software. Therefore, the method of the present invention is more sensitive and can detect an analyte in lower concentrations than the LOCI assay.

In a preferred embodiment, the optical component is a dye. Preferably, the optical component is selected from one of the following fluorescent dyes and mixtures thereof:

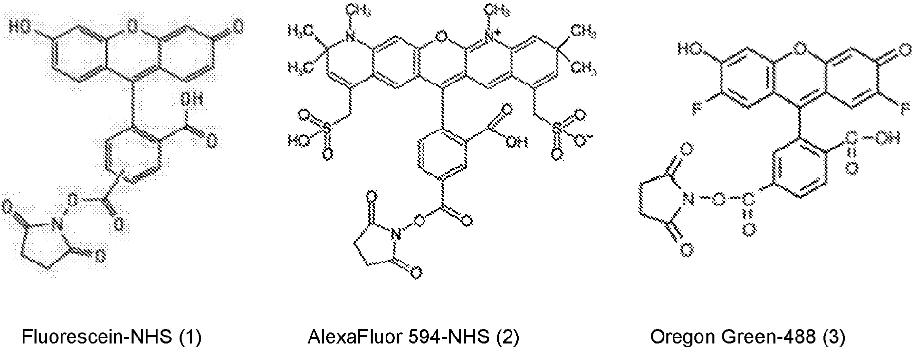

Dyes (1), (2) and (3) are common organic fluorophores that can be attached to proteins and other macromolecules by means of the attached N-hydroxy succinimide groups, which react with amine groups to form a covalent amide bond. These dyes, along with a wide range of other fluorophores can be irreversibly converted into a non-fluorescent state by the method of the present invention.

Preferably, the optical component when in one of the first and second optical states is fluorescent and the optical component when in the other of the first and second optical states is non-fluorescent. In one embodiment, the optical component in the first optical state is fluorescent and in the second optical state is non-fluorescent or the optical component in the first state is non-fluorescent and in the second optical state is fluorescent. More preferably however, the optical component when in the first optical state is fluorescent and the optical component when in the second optical state is non-fluorescent.

Preferably, the change from the first optical state to the second optical state is irreversible. This allows for subsequent scanning of the substrate to identify areas in which the change in optical state has taken place.

Step (iv) of the method of the present invention involves detecting the set of local regions having the second optical state on the substrate. Step (iv) is preferably carried out by image analysis software that can distinguish actual binding events from transient binding events and surface artefacts by analysis of surface intensity and morphology. Step (iv) may be a separate step that is carried out after the optical component has been converted from the first optical state to the second optical state. Alternatively, step (iv) may be carried out during the course of the conversion to the second optical state.

Any method that can successfully identify individual binding events can be used. For example, an initial image of the surface could be taken using the excitation wavelength of the optical component first, followed by irradiation with electromagnetic radiation to excite both the photosensitiser and the optical component, followed by a final step of irradiation with just the excitation wavelength of the optical component.

Alternatively, it may be possible to capture a series of images (video file) of the surface whilst irradiating with electromagnetic radiation that excites both the optical component and the photosensitiser simultaneously. Any combination of optical filters, dichroic mirrors and illumination sequences that allow the binding events to be identified are acceptable.

The second optical state forms a set of local regions on the substrate. Advantageously, local regions having the second optical state can be counted as individual binding events. The method of the present invention is thus suitable for performing a digital assay. However, if there are a large number of binding events such that the majority of the optical component is in the second optical state, the bulk change can be detected.

In a preferred embodiment, the set of local regions having the second optical state on the substrate are detected by counting local regions in the set of local regions having the second optical state on the substrate or by measuring the set of local regions having the second optical state on the substrate as a bulk property. More preferably, the set of local regions having the second optical state on the substrate are detected by counting local regions in the set of local regions having the second optical state on the substrate.

Local regions having the second optical state may need to be above a threshold value corresponding to a background signal, depending on the first and second optical states. For example, the sample may have some background auto-fluorescence but if this is not above the threshold value then it should not impact the signal due to the digital nature of the assay.

The set of local regions having the second optical state on the substrate are typically discrete areas on the substrate. However, some local regions may be excluded from detection owing to their morphology. Local regions corresponding to an individual binding event tend to be uniform and circular but some local regions may be irregular in shape, corresponding to artefacts. Further, some local regions may be larger than others where particles have clumped together, or they may be smaller, where there has only been a transient binding event. Therefore, in a preferred embodiment, only uniform, circular local regions having the second optical state on the substrate are detected.

The set of local regions on the substrate can be detected using simple optical means. In a preferred embodiment, the set of local regions having the second optical state on the substrate is detected using optical microscopy. An optical set-up suitable for detection is shown in Fig. 6. More preferably, the set of local regions having the second optical state on the substrate is detected using wide-field microscopy. Wide-field microscopy is the simplest form of microscopy, whereby the whole sample is illuminated and imaged at the same time, in comparison to more complex techniques such as confocal microscopy where only one single focal spot is illuminated and recorded at a time. The benefits of confocal microscopy are increased contrast by removal of out-of-focus haze and the ability to take a stack of images through the depth of the sample. Super-resolution microscopy techniques are also known, such as photo-activated localization microscopy (PALM or FPALM) and stochastic optical reconstruction microscopy (STORM). These methods have added complexity and cost over simple wide-field methods.

In order to detect the set of local regions on the substrate, a wide-field fluorescent microscope with a light source (for example, an LED) and a photodetector (for example a photomultiplier tube or a camera, such as a CCD) can be used, using excitation and emission filters suitable for the detection of the particular optical component.

The photosensitiser is proximal to the substrate when the binding event has occurred. That is, the photosensitiser is sufficiently close to the surface of the substrate to interact with the excited optical component and convert it from the first optical state to the second optical state on irradiation of the device. The actual distance between the photosensitiser and the surface of the substrate will, however, depend on a number of variables, such as the size and nature of the photosensitiser, the size and nature of the binding component, the reporter reagent and the analyte, and the nature of the sample medium.

The binding component has a binding site which is capable of binding a reporter reagent proportionally to the concentration of the analyte in the sample. The proportionality is important for the functioning of the assay since the binding must be dependent on the concentration of the analyte for any meaningful measure of the concentration of the analyte to be determined. The binding may be directly proportional or indirectly proportional to the concentration of the analyte depending on the type of assay being performed. In the case of a non-competitive assay, e.g. an immunometric assay, the binding is directly proportional to the concentration of the analyte, but for a competitive assay, the binding is indirectly proportional to the concentration of the analyte.

One particular type of competitive assay is presented in which an antibody to the analyte is immobilised on the substrate, and a labelled analogue of the analyte is introduced into the sample. The analyte and labelled analogue of the analyte then "compete" for the antibody on the surface. In the absence of analyte, then the labelled analogue will bind at the maximum possible rate. However, in the presence of analyte, the antibody on the substrate becomes populated with analyte and the rate of binding of the analogue is diminished.

The binding component may be adapted to bind to the analyte, or a complex or derivative of the analyte, in which case the reporter reagent will bind to the binding component in the presence of the analyte, or the complex or derivative of the analyte. In this case, the binding component has a binding site which is capable of binding to the reporter reagent in the presence of the analyte or the complex or derivative of the analyte. The binding is, however, still proportional to the concentration of the analyte.

Alternatively, the binding component may itself be an analogue of the analyte and the reporter reagent binds directly to the binding component (it is an analogue because it is bound to the surface of the substrate either through covalent bonding or non-covalent interactions). In this case, the binding component will compete with the unbound analyte, or an unbound complex or derivative of the analyte, for the binding of the reporter reagent. Accordingly, the binding component will simply be capable of binding to the reporter reagent.

Determining the extent of binding of the reporter reagent to the binding component (either directly or mediated by the analyte/complex or derivative of the analyte) provides a measurement of the concentration of the analyte in the sample. It is customary that the system has been pre-calibrated at the point of manufacture to generate a calibration curve that is used to convert the instrument signal into the measured analyte concentration in the sample.

The assay also requires the presence of a reporter reagent. The reporter reagent comprises a photosensitiser. The photosensitiser is capable of absorbing electromagnetic radiation to interact with the excited optical component. It is this interaction which causes the excited optical component to change from the first optical state to the second optical state.

The photosensitiser may therefore be composed of any material which is capable of interacting with the electromagnetic radiation in this manner. Suitable photosensitisers are known from photodynamic therapy (PDT) as PDT reagents. PDT reagents are used in cancer therapy and dermatology to destroy cells upon illumination (Shafirstein et al., Cancers, 2017, 9, 12; Wan and Lin, Clinical, Cosmetic and Investigational Dermatology, 2014, 7, 145).

Upon irradiation with electromagnetic radiation, PDT reagents are promoted to an excited triplet state. This excited triplet state can interact directly with cellular components, in what is called a Type I process or can interact with oxygen in what is called a Type II process. Both Type I and Type II processes can lead to the formation of ROS. In the Type II process, the predominant product is singlet oxygen, through an intersystem crossing mechanism.

Singlet oxygen is an excited state of oxygen that is highly reactive. It can undergo a number of reactions before decaying, including Diels-Alder type reactions and ene reactions. It also undergoes general oxidation reactions with sulfur and nitrogen containing compounds. The indiscriminate reactivity of singlet oxygen is one of the reasons why it is used in photodynamic therapy.

A wide range of photosensitiser compounds are known, including porphyrins, chlorins (for example pyropheophorbide-a), phthalocyanines and other polyaromatic species (see, for example, Antibody-Directed Phototherapy, Pye et al., Antibodies, 2013, 2, 270).

In one embodiment, the photosensitiser is selected from porphyrins, chlorins, phthalocyanines and other polyaromatic species. In a preferred embodiment, the photosensitiser is a silicon phthalocyanine derivative, such as the example shown below:

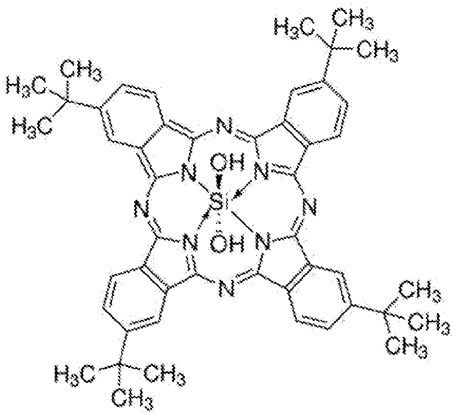

The nature of the binding component and the reporter reagent will depend on the nature of the analyte, but they are preferably antibodies. The method of the present invention has particular applicability in immunoassays. In a particularly preferred embodiment, the binding component is an antibody raised to the analyte or the complex or derivative of the analyte, and the reporter reagent comprises an antibody raised to the analyte or the complex or derivative of the analyte. In principle, a single molecule could be used for each reagent, but in practice, the binding component and the reporter reagent are a population of molecules. The term "antibody" preferably includes within its scope a Fab fragment, a single-chain variable fragment (scFv), and a recombinant binding fragment.

As alternatives to antibody-antigen reactions, the binding component, the reporter reagent and the analyte may be a first and second nucleic acid where the first and second nucleic acids are complementary, or a reagent containing avidin or derivatives thereof and an analyte containing biotin or derivatives thereof, or vice versa. The binding component and the reporter reagent may also be aptamers. The system is also not limited to biological assays and may be applied, for example, to the detection of heavy metals in water. The system also need not be limited to liquids and any fluid system may be used, e.g. the detection of enzymes, cells and viruses etc. in the air.

The maximum observable signal is the maximum signal that can be achieved when monitoring the photosensitiser binding to a surface. The binding of particles to the substrate is governed by the diffusion rate of the analyte and reporter reagent which is, in turn, governed largely by the hydrodynamic radius of these components and the viscosity/temperature of the sample.

The device used in the method of the present invention may further comprise controls which compensate for natural variability in the components of the measuring system, variability in the samples that are measured, and variability in the environmental conditions during the measurement. This can be achieved by exposing the sample to reagents on the surface of the substrate. The different reagents are typically located at different areas of the surface of the substrate, these areas being coated in different reagents. These controls are defined as "negative" and "positive" controls, in the sense that the negative control should approximate the expected signal in the absence of analyte, and the positive control should approximate the expected signal when analyte has saturated the system.

To achieve detection with these controls, the device of the present invention preferably comprises a binding component, a negative control reagent and a positive control reagent, each of which is attached to the surface of the substrate as described above.

The binding component is as described above.

The negative control reagent has a lower affinity for the reporter reagent under the conditions of the assay than the binding component. Accordingly, the negative control reagent provides the negative control. It is important that the affinity is considered under the conditions of the assay. The reason is that in the case of a non-competitive assay, the affinity of the binding component for the reporter reagent is mediated by the presence of the analyte, or the complex or derivative of the analyte. Thus, in the absence of the analyte, or the complex or derivative of the analyte, neither the binding component nor negative control reagent has any affinity for the reporter reagent. However, in the presence of the analyte, or the complex or derivative of the analyte, the negative control reagent has a lower affinity for the reporter reagent than the binding component.

In addition, in the embodiments where the binding component binds to the analyte, or the complex or derivative of the analyte, the negative control reagent preferably has a lower affinity for the analyte or, if used, the complex or derivative of the analyte than the binding component. The negative control reagent is preferably a protein and more preferably an antibody. The negative control reagent typically has similar chemical and physical properties to the binding component, but provides little or no affinity for the reporter reagent under the conditions of the assay. In a particularly preferred embodiment, the negative control reagent has essentially no affinity for the reporter reagent under the conditions of the assay. Preferably, the negative control reagent provides essentially no affinity for the analyte or the complex or derivative of the analyte. That is, the binding of the reporter reagent, or, where applicable, the analyte or the complex or derivative of the analyte, to the negative control reagent is non-specific. In this manner, the negative control reagent can compensate for non-specific binding of the reporter reagent to the binding component. In certain embodiments, a software algorithm uses the data from the negative control region as part of the calculation to give the analyte concentration.

The positive control reagent binds to the reporter reagent and has an affinity for the reporter reagent which is less influenced by the concentration in the sample of the analyte or, if used, the complex or derivative of the analyte than the binding component and hence provides the positive control. Preferably, the positive control reagent has an affinity for the reporter reagent which is essentially independent of the concentration of the analyte or the complex or derivative of the analyte. More preferably, the positive control reagent has a higher affinity for the reporter reagent under the conditions of the assay than the binding component. In this manner, the positive control reagent measures the maximum expected signal in the system.

For both the negative and positive control measurements, a software algorithm may detect unusual binding patterns that could lead to an error message and abort the measurement process.

In order to increase the dynamic range of an assay performed in accordance with the present invention, whilst also improving precision, it is preferred to have the binding component in a plurality of locations on the substrate. These locations may be tuned to different sensitivities, by varying the concentration of the binding component at each location. Each location may also have its own negative control and positive control reagents to act as controls for the different dynamic ranges. This is particularly applicable to competitive assays which are particularly sensitive to the concentration of each of the individual components which make up the system.

Although the description above allows the assay to reach an equilibrium before activating the photosensitiser, it is also possible that the kinetics of the binding events could be monitored by irradiating the photosensitiser for discrete periods of time over a time-course and monitoring the binding events as they take place over the course of the reaction, prior to equilibrium being attained.

The analyte may be a macromolecule or a small molecule. The macromolecule is typically a protein, such as a protein-based hormone, and may also be part of a larger particle, such as a virus, a bacterium, a cell (e.g. a red blood cell) or a prion. The small molecule may be a drug.

The term "small molecule" used herein is a term of the art and is used to distinguish the molecule from macromolecules such as proteins and nucleic acids. A small molecule is often referred to in the field of immunoassays as a "hapten", being a small molecule which, when attached to a large carrier molecule such as a protein, can elicit an immune response and includes molecules such as hormones and synthetic drugs. A small molecule of this type will typically have a molecular weight of 2,000 or less, often 1,000 or less and even 500 or less. The binding component may be adapted to bind to the analyte itself, although the analyte can undergo a chemical reaction or initial complexing event before binding to the binding component. For example, the analyte might be protonated/deprotonated in the pH of the assay conditions. Thus, the analyte which is bound to the binding component may be the analyte itself or a derivative of the analyte; both are included within the scope of the present invention.

In a preferred embodiment, the present invention may be used to detect the presence of multiple analytes in the same sample at the same time. Different binding components can be used in different locations on the substrate for the measurement of each analyte. Sandwich and competitive assays may be run in parallel and the assays may use the same negative and positive controls as described above, or there may be separate controls for each analyte being measured.

The sample which is suspected of containing the analyte of interest will generally be a fluid sample, e.g. a liquid sample, and usually a biological sample, such as a bodily fluid, e.g. blood, plasma, saliva, serum, intraocular fluid, cerebrospinal fluid or urine. The sample may contain suspended particles and may even be whole blood. In a preferred embodiment, the sample is unprocessed and more preferably, is an unprocessed fluid. By unprocessed is meant that the sample/fluid is not pre-treated by filtration, dilution or any other pre-processing step prior to being mixed with the reporter reagent and other assay components. An advantage of the method of the present invention is that the assay may be performed on a sample which contains suspended particles without unduly influencing the results of the assay.

In a preferred embodiment, the sample is whole blood. It is surprising that the components of whole blood do not interfere with the method of detection of the present invention. It is usual to remove the red blood cells from blood to measure fluorescence in the plasma or serum component of the blood due to unpredictable scattering of light by the cellular components, which varies from sample to sample. However, in the method of the present invention, because the fluorescence would be measured on the substrate using an imaging system with a shallow depth of field, and because individual binding events can be measured, the measurement can take place in whole blood.

The sample will typically be in the order of microlitres (e.g. 1-100 µL, preferably 1-10 µL). In order to hold a fluid sample, the substrate is preferably located in a sample chamber having one or more side walls, an upper surface and a lower surface. Accordingly, the device used in the method of the present invention preferably further comprises a chamber for holding the sample containing the analyte in contact with the substrate.

A potential additional source of background interference is the settling of suspended particles on to the surface of the substrate, including reporter reagent and cellular components of the sample. This source of interference may be reduced by positioning the substrate above the bulk solution, e.g. on the upper surface of the reaction chamber. Thus, if any settling occurs, it will not interfere with the substrate. Preferably the substrate forms the upper surface as shown in the figures. Preferably, the substrate is substantially planar. More preferably, the substrate is planar. By "substantially planar" it is meant that the substrate deviates from planarity only up to a point where it remains functional in the invention, for example, such that the whole of the substrate remains within a single focal range when being imaged. Clearly, the optical and binding components will be on the interior surfaces of the chamber to allow contact with the sample. This and other modifications are included in the scope of the present invention.

The sample may simply be retained by surface tension forces, for example, inside a capillary channel.

The reporter reagent and optionally one or more additional reagents are preferably stored in a chamber incorporated into the device used in the method of the present invention.

The method of the present invention is especially useful in point-of-care (POC) testing. POC testing is defined as diagnostic testing at or near the point of care, i.e. bedside testing. POC testing enables convenient and rapid testing, allowing improved decision making and triage whilst better allocating hospital resources such as accident and emergency care and hospital beds. This contrasts with traditional testing in which samples were taken at the point of care and then sent to the laboratory for testing. With such testing, it often takes hours or days to get the results, during which time care must continue without the desired information. POC testing often uses test kits in combination with portable instruments.

The method of the present invention is particularly useful for monitoring the concentration or presence/absence of analytes that are normally in very low abundance. Potential applications including measuring biomarkers in cardiac disease (e.g. high-sensitivity troponin), infectious disease (e.g. Hepatitis C core antigen), aging/dementia (e.g. Alzheimer's disease markers Amyloid Beta and Tau), cytokines and oncology (e.g. circulating tumour markers).

The present invention may also provide a device for detecting an analyte in a sample, the device comprising: a substrate having an optical component and a binding component, wherein the optical component and the binding component are attached to a surface of the substrate, the optical component being capable of being excited in response to irradiation with electromagnetic radiation, and the excited optical component being capable of changing from a first optical state to a second optical state in response to interaction with irradiated photosensitisers of reporter reagents bound to the surface of the substrate in proportion to the concentration of analyte in the sample, thereby to form a set of local regions having the second optical state on the substrate.

The features of the device are as described above for the device used in the method of the present invention.

In a preferred embodiment, the device further comprises a chamber for holding a mixture of the sample and the reporter reagent.

The device may include one or more radiation sources which are adapted to generate electromagnetic radiation and a detector which is adapted to detect the second optical state thereby allowing a precise determination of the position of the photosensitiser with respect to the substrate.

The device may take the form of a cartridge to be used with a separate reader. The reader may incorporate the radiation source(s) and the detector. The reader is preferably a portable reader. Preferably, the device comprises a cartridge, wherein the substrate is within the cartridge, and wherein the device further comprises a detector for detecting the set of local regions having the second optical state on the substrate. The present invention may also provide the cartridge comprising the substrate and the optical and binding components as defined herein. The cartridge is preferably a disposable cartridge.

The present invention may also provide a system for detecting an analyte in a sample, comprising: the device as described above; and the reporter reagent for forming a mixture comprising the sample, the reporter reagent comprising the photosensitiser, wherein the photosensitiser is capable of absorbing electromagnetic radiation to interact with the excited optical component to change the excited optical component from the first optical state to the second optical state.

Preferably, the photosensitiser is capable of absorbing electromagnetic radiation to interact with a pre-activator reagent present in the mixture to generate an activator reagent, and the activator reagent is capable of interacting with the excited optical component to change the excited optical component from the first optical state to the second optical state.

In a preferred embodiment, the system of the present invention consists essentially of the above-described features. By "essentially" is meant that no other features are required to perform the assay.

The present invention will now be described with reference to the following examples which are not intended to be limiting. These examples are simplified to have direct binding of the reporter reagent to the binding component. However, it is to be understood that a variety of assay formats are envisaged where the binding of the reporter reagent to the binding component is either promoted or inhibited by the presence of analyte in solution. The examples are given to demonstrate the mode of conversion of the excited optical component from the first optical state to the second optical state.

### Examples

### Materials

Poly-lysine hydrochloride (MW30kD), Innolink biotin 354S, biotin-NHS ester, Silicon 2,9,16,23-tetra-tert-butyl-29H,31H-phthalocyanine dihydroxide, goat anti-mouse antibody and other general lab reagents were sourced from Merck Millipore.

AlexaFluor 594 Streptavidin conjugate and Alexafluor Oregon Green 488 conjugate were sourced from Thermo Fisher.

Carboxy polystyrene particles (100nm diameter) were sourced from Bangs Laboratories.

5409 anti-TSH monoclonal antibody was sourced from Medix Biochemica.

Biotinylated poly-lysine hydrochloride (5 biotins per poly-lysine) was prepared by the reaction of Innolink biotin 354S with poly-lysine by methods known in the art. The biotin incorporation was measured by absorbance at 354nm. Biotinylation of 5409 antibody was carried out by methods known in the art. The biotin incorporation (approximately two) was measured by the HABA assay.

Carboxy polystyrene particles were infused with silicon phthalocyanine reagent using methods described by Ullman *et al* (Ullman et al, Clinical Chemistry, 1996, 42, 1518-1526). Goat anti-mouse antibody was covalently attached to these particles using EDC/NHS coupling reagents by methods known in the art.

### Example 1 - preparation of AlexaFluor 594 substrate chip

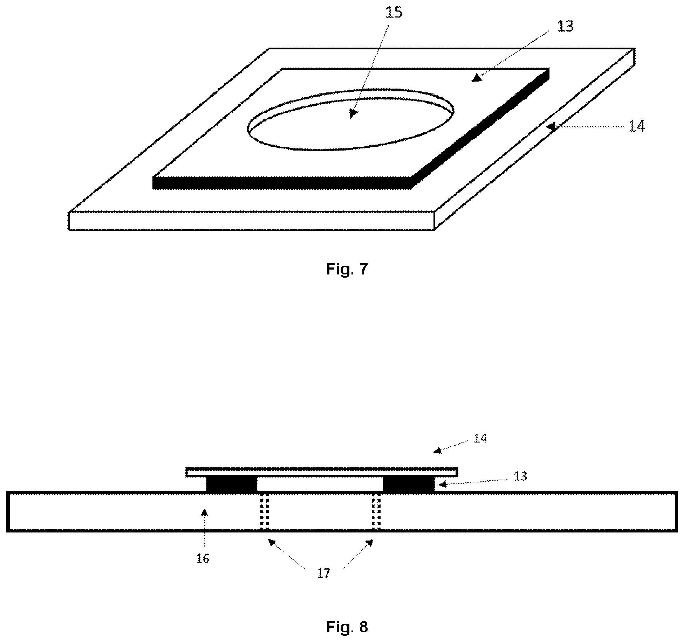

A 22 mm glass cover slip (Brand GMBH) was cleaned using a Henniker Cirrus atmospheric plasma etcher to remove surface impurities from the glass. 50 microlitres of biotin-poly-lysine conjugate (at 50 micrograms / mL in deionised water) was then deposited on the surface of the cover slip for 10 minutes, then washed with deionised water and dried. A 1 cm by 1 cm square piece of 200 µm thickness pressure sensitive adhesive 13 with a 6 mm diameter hole cut out, was then attached to the cover slip to create a shallow well 15 as shown in Fig. 7.

30 microlitres of streptavidin AlexaFluor 594 conjugate (10 micrograms / mL) was then incubated in the shallow well for 30 minutes, then washed off and replaced with 30 microlitres of biotinylated 5409 antibody for an additional 30 minutes. The surface was washed several times with phosphate buffer containing 0.05% BSA and 2% sucrose, then dried in air at room temperature.

The release liner was then removed from the PSA, the substrate was inverted and attached to a piece of acrylic sheet 16 with two small holes 17 drilled through to create a reaction chamber, as shown in profile in Fig. 8.

### Example 2 - preparation of Oregon Green 488 substrate chip

This surface was prepared as described in Example 1, replacing the AlexaFluor Streptavidin conjugate with Oregon Green 488 streptavidin conjugate at the same concentration.

### Example 3 - incubation of beads with AlexaFluor 594 substrate or Oregon Green 488 substrate

To the reaction chambers from Examples 1 and 2 were added 8 microlitres of a 1 :2000 dilution of photosensitiser beads coated in goat anti-mouse antibody (beads at 0.5% solids) in phosphate buffer 0.05% BSA, 0.05% Tween-20 under low-lighting conditions. The two holes in the plastic cartridge were sealed with clear adhesive tape and incubated for 30 minutes.

### Example 4 - illumination and imaging of the AlexaFluor 594 substrate

A number of chips were activated and imaged using an in-house prototype instrument as shown in Fig. 5. The instrument has two high-powered LED light sources that can each be focussed onto the substrate surface through a 63X objective lens. The 680 nm red LED is centred on the excitation wavelength of the photosensitiser beads and the 560 nm LED coincides with the excitation wavelength of the AlexaFluor 594 fluorophore. Each LED can be controlled separately and the drive current (and hence light intensity) can be adjusted separately. The maximum drive current for continuous use of the 680 nm LED is 0.6 A and for the 560 nm LED the maximum drive current is 1.0 A. Images are collected on a camera chip that is connected to a PC that controls the focus, exposure and gain of the camera.

Four data sets were collected. For each data set, the surface was illuminated with either the 680 nm LED, the 560 nm LED or both LEDs. Images were taken of the surface at a number of discrete time points. The images were taken of the surface using illumination with only the 560 nm LED. Dark spots started to form over time under the different illumination conditions. For each captured image, the diameter of a number of representative spots were measured using Image J software to obtain the average size of the spots at that particular timepoint. The size of the spots is given in pixel counts, where 20 pixels is approximately 1 micron.

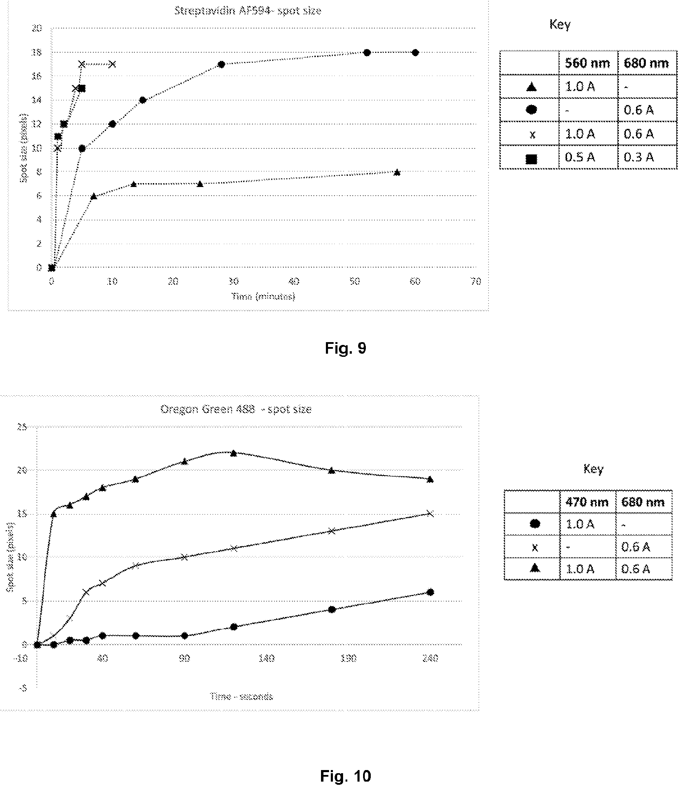

As can be seen in Fig. 9, the rate of spot formation varies, depending upon the illumination conditions. When illuminated solely at 560 nm (1.0 A), the spots reach a size of approximately 6 pixels after 10 minutes and a maximum size of around 8 pixels after approximately 1 hour. When illuminating with just 680 nm light (0.6 A), the spot formation is more rapid, reaching 10 pixels in 6 minutes and reaching a maximum of 18 pixels after approximately 1 hour. When illuminating with both wavelengths simultaneously, spot formation is more rapid, reaching 10 pixels in 1 minute. Additionally, when the drive current in the LEDs is reduced by to 0.5 A and 0.3 A for the 560 nm and 680 nm LEDs respectively, the rate of spot formation remains approximately the same. The photosensitiser has low absorption at 560 nm, so it was surprising that spots formed with just this wavelength. However, little effort was made to eliminate ambient light and / or straylight passing through the filters, so it is presumed that there was sufficient light to partially activate the beads. What can be seen clearly is the rate of spot formation is enhanced when using both light sources versus using them independently. Additionally, the intensity of each light source can be lowered without affecting spot formation when both LEDs are used.

### Example 5 - illumination and imaging of the Oregon Green 488 substrate

The experimental setup was the same as for Example 4, except a 470 nm LED was used in place of the 560 nm LED. The 470 nm LED had a maximum drive current of 1.0 A for continuous use. The results are shown in Fig. 10. Oregon Green 488 is less photostable compared to AlexaFluor 594, and the rate of spot formation is also more rapid. Again, spots could be formed by solely using the 470 nm LED, although these were very small. When illuminating with the 680 nm LED on its own, spot formation was relatively rapid, reaching 10 pixels in around 90 seconds. When both LEDs were used simultaneously, spot formation was extremely rapid, occurring within a few seconds and spots of diameter greater than 15 pixels were formed in less than 20 seconds.

## Claims

1. A method for detecting an analyte in a sample, the method comprising the steps of:
(i) providing a mixture comprising a sample and a reporter reagent (2) to a device, wherein the reporter reagent (2) comprises a photosensitiser (1), the device comprising a substrate (11) having an optical component (4) and a binding component (3), wherein the optical component (4) and the binding component (3) are attached to a surface of the substrate (11);
(ii) allowing a portion of the reporter reagent (2) to bind to the surface of the substrate (11) in proportion to the concentration of the analyte, by means of the binding component (3);
(iii) irradiating the device with electromagnetic radiation for absorption by the optical component (4) and for absorption by the photosensitiser (1) of the reporter reagent (2), such that the optical component (4) absorbs electromagnetic radiation to generate an excited optical component, and the photosensitiser (1) of the reporter reagent (2) bound to the surface of the substrate (11) absorbs electromagnetic radiation and interacts with the excited optical component to cause the excited optical component to change from a first optical state (4) to a second optical state (5), thereby forming a set of local regions having the second optical state (5) on the substrate (11); and
(iv) detecting the set of local regions having the second optical state (5) on the substrate (11).

2. A method as claimed in claim 1, wherein the electromagnetic radiation for absorption by the optical component (4) and for absorption by the photosensitiser (1) of the reporter reagent (2) have the same wavelength.

3. A method as claimed in claim 1, wherein the electromagnetic radiation for absorption by the optical component (4) and for absorption by the photosensitiser (1) of the reporter reagent (2) have different wavelengths.

4. A method as claimed in claim 3, wherein the electromagnetic radiation for absorption by the optical component (4) and for absorption by the photosensitiser (1) of the reporter reagent (2) arrives at the device from one direction.

5. A method as claimed in claim 3, wherein the electromagnetic radiation for absorption by the optical component (4) and for absorption by the photosensitiser (1) of the reporter reagent (2) arrives at the device from different directions.

6. A method as claimed in any one of claims 3 to 5, wherein the device is irradiated with electromagnetic radiation for absorption by the optical component (4) and for absorption by the photosensitiser (1) of the reporter reagent (2) simultaneously, or wherein the device is irradiated first with electromagnetic radiation for absorption by the photosensitiser (1) of the reporter reagent (2) and secondly with electromagnetic radiation for absorption by the optical component (4).

7. A method as claimed in any preceding claim, wherein the photosensitiser (1) of the reporter reagent (2) absorbs electromagnetic radiation and interacts with a pre-activator reagent present in the mixture to generate an activator reagent which interacts with the excited optical component to cause the excited optical component to change from the first optical state (4) to the second optical state (5).

8. A method as claimed in claim 7, wherein the activator reagent is a reactive oxygen species.

9. A method as claimed in claim 8, wherein the reactive oxygen species is singlet oxygen.

10. A method as claimed in any preceding claim, wherein the first optical state (4) is fluorescent and the second optical state is non-fluorescent (5).

11. A method as claimed in any preceding claim, wherein the change from the first optical state (4) to the second optical state (5) is irreversible.

12. A method as claimed in any preceding claim, wherein steps (i) to (iii) take place in the absence of wash steps.

13. A method as claimed in any preceding claim, wherein the sample is unprocessed.

14. A method as claimed in any preceding claim, wherein the device is irradiated with electromagnetic radiation for more than 1 second.

## Patentansprüche

1. Verfahren zum Nachweisen eines Analyten in einer Probe, wobei das Verfahren die folgenden Schritte umfasst:
(i) Bereitstellen eines Gemisches, das eine Probe und ein Reporterreagens (2) umfasst, für eine Vorrichtung, wobei das Reporterreagens (2) einen Photosensibilisator (1) umfasst, wobei die Vorrichtung ein Substrat (11) mit einer optischen Komponente (4) und einer Bindungskomponente (3) umfasst, wobei die optische Komponente (4) und die Bindungskomponente (3) an eine Oberfläche des Substrats (11) gebunden sind;
(ii) Ermöglichen, dass ein Abschnitt des Reporterreagens (2) mittels der Bindungskomponente (3) proportional zu der Konzentration des Analyten an die Oberfläche des Substrats (11) bindet;
(iii) Bestrahlen der Vorrichtung mit elektromagnetischer Strahlung zur Absorption durch die optische Komponente (4) und zur Absorption durch den Photosensibilisator (1) des Reporterreagens (2), sodass die optische Komponente (4) elektromagnetische Strahlung absorbiert, um eine angeregte optische Komponente zu erzeugen, und der Photosensibilisator (1) des Reporterreagens (2), der an die Oberfläche des Substrats (11) gebunden ist, elektromagnetische Strahlung absorbiert und mit der angeregten optischen Komponente interagiert, um zu bewirken, dass die angeregte optische Komponente von einem ersten optischen Zustand (4) in einen zweiten optischen Zustand (5) wechselt, wodurch ein Satz lokaler Regionen mit dem zweiten optischen Zustand (5) auf dem Substrat (11) gebildet wird; und
(iv) Nachweisen des Satzes lokaler Regionen mit dem zweiten optischen Zustand (5) auf dem Substrat (11).

2. Verfahren nach Anspruch 1, wobei die elektromagnetische Strahlung zur Absorption durch die optische Komponente (4) und zur Absorption durch den Photosensibilisator (1) des Reporterreagens (2) die gleiche Wellenlänge aufweisen.

3. Verfahren nach Anspruch 1, wobei die elektromagnetische Strahlung zur Absorption durch die optische Komponente (4) und zur Absorption durch den Photosensibilisator (1) des Reporterreagens (2) unterschiedliche Wellenlängen aufweisen.

4. Verfahren nach Anspruch 3, wobei die elektromagnetische Strahlung zur Absorption durch die optische Komponente (4) und zur Absorption durch den Photosensibilisator (1) des Reporterreagens (2) aus einer einzigen Richtung zu der Vorrichtung gelangt.

5. Verfahren nach Anspruch 3, wobei die elektromagnetische Strahlung zur Absorption durch die optische Komponente (4) und zur Absorption durch den Photosensibilisator (1) des Reporterreagens (2) aus verschiedenen Richtungen zu der Vorrichtung gelangt.

6. Verfahren nach einem der Ansprüche 3 bis 5, wobei die Vorrichtung gleichzeitig mit elektromagnetischer Strahlung zur Absorption durch die optische Komponente (4) und zur Absorption durch den Photosensibilisator (1) des Reporterreagens (2) bestrahlt wird, oder wobei die Vorrichtung zunächst mit elektromagnetischer Strahlung zur Absorption durch den Photosensibilisator (1) des Reporterreagens (2) und danach mit elektromagnetischer Strahlung zur Absorption durch die optische Komponente (4) bestrahlt wird.

7. Verfahren nach einem der vorhergehenden Ansprüche, wobei der Photosensibilisator (1) des Reporterreagens (2) elektromagnetische Strahlung absorbiert und so mit einem in dem Gemisch vorhandenen Präaktivatorreagens wechselwirkt, dass ein Aktivatorreagens erzeugt wird, das mit der angeregten optischen Komponente wechselwirkt, um zu bewirken, dass die angeregte optische Komponente von dem ersten optischen Zustand (4) in den zweiten optischen Zustand (5) wechselt.

8. Verfahren nach Anspruch 7, wobei das Aktivatorreagens eine reaktive Sauerstoffspezies ist.

9. Verfahren nach Anspruch 8, wobei die reaktive Sauerstoffspezies Singulett-Sauerstoff ist.

10. Verfahren nach einem vorhergehenden Anspruch, wobei der erste optische Zustand (4) fluoreszierend ist und der zweite optische Zustand nicht fluoreszierend (5) ist.

11. Verfahren nach einem vorhergehenden Anspruch, wobei der Wechsel von dem ersten optischen Zustand (4) zu dem zweiten optischen Zustand (5) unumkehrbar ist.

12. Verfahren nach einem vorhergehenden Anspruch, wobei die Schritte (i) bis (iii) in Abwesenheit von Waschschritten stattfinden.

13. Verfahren nach einem vorhergehenden Anspruch, wobei die Probe unverarbeitet ist.

14. Verfahren nach einem vorhergehenden Anspruch, wobei die Vorrichtung länger als 1 Sekunde mit elektromagnetischer Strahlung bestrahlt wird.

## Revendications

1. Procédé de détection d'un analyte dans un échantillon, le procédé comprenant les étapes de :
(i) fourniture d'un mélange comprenant un échantillon et un réactif rapporteur (2) à un dispositif, dans lequel le réactif rapporteur (2) comprend un photosensibilisateur (1), le dispositif comprenant un substrat (11) comportant un composant optique (4) et un composant de liaison (3), dans lequel le composant optique (4) et le composant de liaison (3) sont fixés à une surface du substrat (11) ;
(ii) autorisation à une partie du réactif rapporteur (2) de se lier à la surface du substrat (11) proportionnellement à la concentration de l'analyte, au moyen du composant de liaison (3) ;
(iii) irradiation du dispositif avec un rayonnement électromagnétique destiné à être absorbé par le composant optique (4) et destiné à être absorbé par le photosensibilisateur (1) du réactif rapporteur (2), de sorte que le composant optique (4) absorbe un rayonnement électromagnétique pour générer un composant optique excité, et le photosensibilisateur (1) du réactif rapporteur (2) lié à la surface du substrat (11) absorbe un rayonnement électromagnétique et interagit avec le composant optique excité pour faire passer le composant optique excité d'un premier état optique (4) à un second état optique (5), formant ainsi un ensemble de régions locales comportant le second état optique (5) sur le substrat (11) ; et
(iv) détection de l'ensemble de régions locales comportant le second état optique (5) sur le substrat (11).

2. Procédé selon la revendication 1, dans lequel le rayonnement électromagnétique destiné à être absorbé par le composant optique (4) et destiné à être absorbé par le photosensibilisateur (1) du réactif rapporteur (2) comportent la même longueur d'onde.

3. Procédé selon la revendication 1, dans lequel le rayonnement électromagnétique destiné à être absorbé par le composant optique (4) et destiné à être absorbé par le photosensibilisateur (1) du réactif rapporteur (2) comportent des longueurs d'onde différentes.

4. Procédé selon la revendication 3, dans lequel le rayonnement électromagnétique destiné à être absorbé par le composant optique (4) et destiné à être absorbé par le photosensibilisateur (1) du réactif rapporteur (2) arrive au dispositif en provenance d'une direction.

5. Procédé selon la revendication 3, dans lequel le rayonnement électromagnétique destiné à être absorbé par le composant optique (4) et destiné à être absorbé par le photosensibilisateur (1) du réactif rapporteur (2) arrive au dispositif en provenance de directions différentes.

6. Procédé selon l'une quelconque des revendications 3 à 5, dans lequel le dispositif est irradié avec un rayonnement électromagnétique destiné à être absorbé par le composant optique (4) et destiné à être absorbé par le photosensibilisateur (1) du réactif rapporteur (2) simultanément, ou dans lequel le dispositif est irradié en premier lieu avec un rayonnement électromagnétique destiné à être absorbé par le photosensibilisateur (1) du réactif rapporteur (2) et en second lieu avec un rayonnement électromagnétique destiné à être absorbé par le composant optique (4).

7. Procédé selon une quelconque revendication précédente, dans lequel le photosensibilisateur (1) du réactif rapporteur (2) absorbe un rayonnement électromagnétique et interagit avec un réactif pré-activateur présent dans le mélange pour générer un réactif activateur qui interagit avec le composant optique excité pour faire passer le composant optique excité du premier état optique (4) au second état optique (5).

8. Procédé selon la revendication 7, dans lequel le réactif activateur est une espèce oxygène réactive.

9. Procédé selon la revendication 8, dans lequel l'espèce d'oxygène réactive est l'oxygène singulet.

10. Procédé selon une quelconque revendication précédente, dans lequel le premier état optique (4) est fluorescent et le second état optique est non fluorescent (5).

11. Procédé selon une quelconque revendication précédente, dans lequel le passage du premier état optique (4) au second état optique (5) est irréversible.

12. Procédé selon une quelconque revendication précédente, dans lequel les étapes (i) à (iii) ont lieu en l'absence d'étapes de lavage.

13. Procédé selon une quelconque revendication précédente, dans lequel l'échantillon est non transformé.

14. Procédé selon une quelconque revendication précédente, dans lequel le dispositif est irradié avec un rayonnement électromagnétique pendant plus de 1 seconde.
